(19) Europäisches Patentamt
European Patent Office
Office européen des brevets

(11) **EP 2 853 934 A1**

(12) **EUROPÄISCHE PATENTANMELDUNG**

(43) Veröffentlichungstag:
**01.04.2015 Patentblatt 2015/14**

(51) Int Cl.:
**G02B 21/02** *(2006.01)* **G02B 21/22** *(2006.01)*

(21) Anmeldenummer: **14184931.5**

(22) Anmeldetag: **16.09.2014**

(84) Benannte Vertragsstaaten:
**AL AT BE BG CH CY CZ DE DK EE ES FI FR GB
GR HR HU IE IS IT LI LT LU LV MC MK MT NL NO
PL PT RO RS SE SI SK SM TR**
Benannte Erstreckungsstaaten:
**BA ME**

(30) Priorität: **26.09.2013 DE 102013219383**

(71) Anmelder: **Carl Zeiss Meditec AG
07745 Jena (DE)**

(72) Erfinder:
• **Merz, Franz
73433 Aalen (DE)**
• **Högele, Artur
73447 Oberkochen (DE)**

(74) Vertreter: **Carl Zeiss AG - Patentabteilung
Carl-Zeiss-Strasse 22
73447 Oberkochen (DE)**

(54) **Optisches Abbildungssystem**

(57)    Optisches Abbildungssystem (1) zur Erzeugung eines Bildes einer Objektebene mit einem Linsensystem, das ein Hauptobjektiv (20) und eine Reduzieroptik vor dem Hauptobjektiv (20) umfasst und das entlang einer optischen Achse 23 ausgerichtet ist. Die Reduzieroptik weist eine erste Linse (22) mit einer positiven Brechkraft (22) und eine zweite Linse (21) mit einer negativen Brechkraft auf. Eine objektseitige erste Hauptebene (H) und eine bildseitige zweite Hauptebene (H') sind durch das Linsensystem definiert. Durch das optische Abbildungssystem ist ein Beobachtungsstrahlengang definiert, der derart durch das Linsensystem geführt ist, dass der Beobachtungsstrahlengang in der ersten Hauptebene (H) und in der zweiten Hauptebene (H') jeweils einen Abstand B zur optischen Achse des Linsensystems aufweist.

Erfindungsgemäß ist die erste Linse (22) aus einem ersten Material gefertigt, das eine erste Abbezahl aufweist, und die zweite Linse (21) ist aus einem zweiten Material gefertigt ist, das eine zweite Abbezahl aufweist, wobei die erste Abbezahl größer ist als die zweite Abbezahl. Das Linsensystem ist derart ausgestaltet, dass für einen Wellenlängenbereich $\lambda$ von 480nm $\leq \lambda \leq$ 660nm und für eine Hauptwellenlänge e = 546nm folgende Beziehung erfüllt ist:

$$\left| \arctan\left( \frac{B}{f_{e}' + (f_{e} \cdot f_{e}' / (f_{\lambda} - f_{e}))} \right) \right| < 0.5' ,$$

wobei gilt:
$f_e$ = objektseitige Brennweite für die Hauptwellenlänge e bezüglich der ersten Hauptebene (H);
$f_x$ = objektseitige Brennweite für die Wellenlänge $\lambda$ bezüglich der ersten Hauptebene (H);
$f_e'$ = bildseitige Brennweite für die Hauptwellenlänge e bezüglich der zweiten Hauptebene (H').

FIG.1

**Beschreibung**

Die Erfindung betrifft ein optisches Abbildungssystem, insbesondere ein Mikroskop, zur Erzeugung eines Bildes einer Objektebene mit einem Linsensystem, das ein Hauptobjektiv und eine Reduzieroptik zwischen dem Hauptobjektiv und der Objektebene umfasst und das entlang einer optischen Achse ausgerichtet ist. Die Reduzieroptik weist eine erste Linse mit einer positiven Brechkraft und eine zweite Linse mit einer negativen Brechkraft auf. Durch das Linsensystem ist eine objektseitige erste Hauptebene und eine bildseitige zweite Hauptebene definiert. Durch das optische Abbildungssystem ist ein Beobachtungsstrahlengang definiert, der derart durch das Linsensystem geführt ist, dass der Beobachtungsstrahlengang in der ersten Hauptebene und in der zweiten Hauptebene jeweils einen Abstand zur optischen Achse des Linsensystems aufweist.

Bei der Beobachtung eines Objektes durch ein optisches Abbildungssystem, insbesondere ein Stereo-Operationsmikroskop, kann zwischen dem Hauptobjektiv des optischen Abbildungssystems und dem zu beobachtenden Objekt, beispielsweise ein Auge, eine Weitwinkel-Optik in den Strahlengang eingebracht werden. Dies ermöglicht eine Beobachtung des Augenhintergrundes. Zusätzlich zu dieser Weitwinkel-Optik kann eine Reduzieroptik in Strahlengang zwischen der Weitwinkel-Optik und dem Hauptobjektiv des optischen Abbildungssystems eingeschwenkt werden, um die Adaption der Weitwinkel-Optik an ein optisches Abbildungssystem, beispielsweise ein Operationsmikroskop, zu ermöglichen.

Aus der EP 1 227 355 B1 ist ein Mikroskop zur Weitwinkel-Betrachtung Augenoperationen bekannt, das durch wahlweise zuschaltbare Optiken ermöglicht, von dem Augenhintergrund ein Bild zu erzeugen. Das Mikroskop umfasst ein Linsensystem, das ein Hauptobjektiv und vor dem Hauptobjektiv angeordnete Linsen aufweist.

Dieses Mikroskop hat den Nachteil, dass die Abbildungsqualität bei der Weitwinkel-Betrachtung nicht optimal ist. Durch den geringen Abstand des Hauptobjektivs zum zu beobachtenden Objekt ergeben sich für eine in den Strahlengang zuschaltbare Weitwinkeloptik sehr kleine Brennweiten. Durch eine weitere, in den Strahlengang einbringbare Reduzieroptik kann eine Anpassung der Weitwinkeloptik an das Mikroskop erfolgen, jedoch mit dem Nachteil einer reduzierten Abbildungsqualität.

Zusätzlich zu den optischen Nachteilen weist das optische Abbildungssystem gemäß EP 1 227 355 B1 eine relativ große Baulänge auf. Um den Anwender, beispielsweise einen Chirurgen, bei seiner Arbeit mit dem optischen Abbildungssystem am Objekt, beispielsweise einem Patientenauge, nicht unnötig zu behindern, sollte eine Reduzieroptik nur eine geringe Baulänge aufweisen und dazu möglichst nahe am Hauptobjektiv angeordnet sein.

Aufgabe der Erfindung ist es, ein optisches Abbildungssystem bereitzustellen, bei dem unter Verwendung einer Reduzieroptik vor einem Hauptobjektiv eine sehr gute Abbildungsqualität erreicht wird. Ferner ist es eine Aufgabe, ein Abbildungssystem mit einer geringen Baulänge bereitzustellen.

Erfindungsgemäß wird die Aufgabe dadurch gelöst, dass die erste Linse der Reduzieroptik aus einem ersten Material gefertigt ist, das eine erste Abbezahl aufweist, und dass die zweite Linse der Reduzieroptik aus einem zweiten Material gefertigt ist, das eine zweite Abbezahl aufweist, wobei die erste Abbezahl größer ist als die zweite Abbezahl. Dabei ist das Linsensystem derart ausgestaltet, dass für einen Wellenlängenbereich $\lambda$ von $480nm \le \lambda \le 660nm$ und für eine Hauptwellenlänge e = 546nm folgende Beziehung erfüllt ist:

$$\left|\arctan\left(\frac{B}{fe' + (fe \cdot fe' / (f_\lambda - fe))}\right)\right| < 0.5',$$

wobei gilt:

B = Abstand eines Beobachtungsstrahlengangs zur optischen Achse in der ersten Hauptebene H;
fe = objektseitige Brennweite für die Hauptwellenlänge e bezüglich der ersten Hauptebene H;
$f_\lambda$ = objektseitige Brennweite für die Wellenlänge $\lambda$ bezüglich der ersten Hauptebene H;
fe' = bildseitige Brennweite für die Hauptwellenlänge e bezüglich der zweiten Hauptebene H'.

Die Einheit des Terms "0.5' " ist Winkelminuten.

Ist das Linsensystem derart ausgestaltet, dass für alle Wellenlängen $\lambda$ zwischen 480nm und 660nm und eine Hauptwellenlänge = 546nm folgende Bedingung erfüllt ist,

$$\left|\arctan\left(\frac{B}{fe' + (fe \cdot fe' / (f_\lambda - fe))}\right)\right| < 0.5',$$

so ist die Abbildungsqualität für einen Beobachtungsstrahlengang so gut, dass komrastmindernde und störende Bildfehler korrigiert sind, so dass ein gleichbleibender guter Kontrast der Abbildung und eine gleichbleibende gute Bildqualität über den gesamten Wellenlängenbereich λ erreicht werden.

In einer Ausgestaltung der Erfindung sind das erste Material und das zweite Material derart gewählt, dass eine Differenz der ersten Abbezahl und der zweiten Abbezahl zwischen 16 und 22 liegt.

Bei der Gestaltung des Linsensystems hat es sich als besonders vorteilhaft erwiesen, das erste Material der Linse mit der positiven Brechkraft und das zweite Material der Linse mit der negativen Brechkraft so zu wählen, dass die Differenz der Abbezahlen der beiden Materialien zwischen 16 und 22 liegt. Damit kann die beschriebene Bedingung gut erfüllt werden und es ergibt sich vorteilhaft ein sehr guter Kontrast über den gesamten Wellenlängenbereich bei einer geringen chromatischen Winkelabweichung. Besonders vorteilhaft wird die Linse mit der positiven Brechkraft aus einem Material mit hoher Abbezahl und für die Linse mit der negativen Brechkraft aus einem Material mit niedriger Abbezahl gebildet, wobei die Differenz der Abbezahlen zwischen 16 und 22 liegt.

In einer weiteren Ausgestaltung der Erfindung sind das erste Material und das zweite Material derart gewählt, dass eine erste Brechzahl des ersten Materials größer ist als 1,6 und eine zweite Brechzahl des zweiten Materials größer ist als 1,6.

Durch die Verwendung von Materialien mit hohen rechzahlen, d. h. größer als 1.6, für die erste und zweite Linse der Reduzieroptik kann die obige Bedingung gut erfüllt werden. Damit ergibt sich vorteilhaft ein gut korrigiertes Bild mit sehr gutem Kontrast und einer geringen chromatischen Winkelabweichung.

In einer weiteren Ausgestaltung der Erfindung ist die erste Linse fest angeordnet und die zweite Linse ist verschiebbar in Richtung der optischen Achse angeordnet.

Die zu beobachtende Objektebene kann in der Entfernung von dem Hauptobjektiv variieren. Damit kann es notwendig sein, den Fokus der optischen Beobachtungseinrichtung an die geänderte Objektebene anzupassen. Damit die Fokuseinstellung des Mikroskops unverändert bleiben kann, ist es vorteilhaft, wenn die Reduzieroptik die Möglichkeit einer Fokussierung bietet. Diese Fokussiermöglichkeit lässt sich relativ einfach dadurch erreichen, indem die erste Linse der Reduzieroptik fest angeordnet ist und die zweite Linse eine Relativbewegung entlang der optischen Achse ausführen kann. Durch die Erfüllung der Bedingung im Anspruch 1 ist über den gesamten Fokussierbereich eine gute Korrektur der chromatischen Winkelabweichung gewährleistet.

In einer weiteren Ausgestaltung der Erfindung ist die zweite Linse fest angeordnet und die erste Linse ist verschiebbar in Richtung der optischen Achse angeordnet.

Die gleichen Vorteile, die in der vorherigen Ausgestaltung beschrieben sind, lassen sich erreichen, wenn die zweite Linse der Reduzieroptik fest angeordnet ist und die erste Linse der Reduzieroptik verschiebbar in Richtung der optischen Achse angeordnet ist.

In einer weiteren Ausgestaltung der Erfindung ist die Reduzieroptik in den Strahlengang vor dem am Hauptobjektiv einschwenkbar.

Um dem Anwender eines Mikroskops zu ermöglichen, variabel mit oder ohne Reduzieroptik zu arbeiten, ist es vorteilhaft, wenn die Reduzieroptik einfach in den Strahlengang eingebracht oder aus dem Strahlengang entfernt werden kann. Dadurch kann der Anwender schnell und einfach zwischen zwei Fokusebenen hin- und herschalten, ohne die Fokuseinstellung des Mikroskops verändern zu müssen. Vorteilhaft lässt sich die Reduzieroptik sehr einfach und schnell durch eine Schwenkvorrichtung in den Strahlengang vor dem Hauptobjektiv ein- und ausschwenken.

In einer weiteren Ausgestaltung der Erfindung ist vor der Reduzieroptik ein weiteres optisches Element zur Erzeugung eines Zwischenbildes im Beobachtungsstrahlengang angebracht und das optische Abbildungssystem ist auf das Zwischenbild fokussiert.

Vor der Reduzieroptik kann ein weiteres optisches Element in den Strahlengang eingebracht sein. Die beobachtete Objektebene kann dann eine Zwischenbildebene darstellen, die sich räumlich zwischen dem weiteren optischen Element und der Reduzieroptik im Strahlengang ergibt. Mängel in der Abbildungsqualität, die durch das Einbringen des weiteren optischen Elementes entstehen, lassen sich besonders vorteilhaft durch die Reduzieroptik chromatisch korrigieren, so dass ein sehr gutes, kontrastreiches Bild ohne Bildversatz beobachtet werden kann.

In einer weiteren Ausgestaltung der Erfindung ist das optische Abbildungssystem als ein Stereomikroskop ausgebildet, das einen ersten Beobachtungsstrahlengang und einen zweiten Beobachtungsstrahlengang aufweist, wobei der erste und der zweite Beobachtungsstrahlengang in der ersten Hauptebene H und in der zweiten Hauptebene H' jeweils einen Abstand B zur optischen Achse des Linsensyste aufweisen.

Die Linsen der Reduzieroptik sind üblicherweise rotationssymmetrisch bezüglich der optischen Achse ausgeführt. Dadurch, dass die optische Achse des Hauptobjektivs und die optische Achse der Reduzieroptik identisch sind, ist die optische chromatische Korrektur für alle Beobachtungsstrahlengänge, die in einem Abstand B zur optischen Achse bezüglich der beiden Hauptebenen H und H' verlaufen, gleich gut. Für eine Stereomikroskop ergibt sich dadurch der Vorteil, dass durch eine einzige Reduzieroptik beide Beobachtungsstrahlengänge chromatisch gleich gut korrigiert sind. Dieser Vorteil gilt auch für jeden weiteren Beobachtungsstrahlengang, der in einem Abstand B zur optischen Achse bezüglich der beiden Hauptebenen H und H' geführt ist.

Weitere Vorteile und Merkmale der Erfindung werden in Bezug auf die nachfolgenden Zeichnungen erklärt, in welchen zeigen:

Figur 1    ein erstes Ausführungsbeispiel eines erfindungsgemäßen optischen Abbildungssystems mit einer vor einem Hauptobjektiv angebrachten Reduzieroptik;

Figur 2    eine schematische Darstellung eines Strahlengangs in einem optischen Abbildungssystem gemäß Figur 1;

Figur 3    eine bildseitige chromatische Winkelabweichung bezogen auf eine Wellenlänge zwischen 480nm und 660nm für drei unterschiedliche Brennweiten des optischen Abbildungssystems gemäß Figur 1.

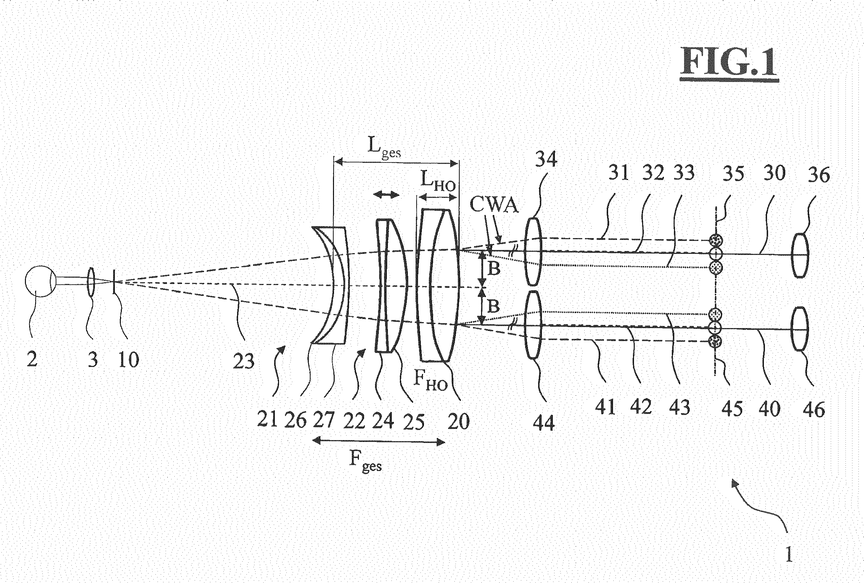

In Figur 1 ist ein Ausführungsbeispiel eines erfindungsgemäßen optischen Abbildungssystems 1 mit einer vor einem Hauptobjektiv 20 angebrachten Reduzieroptik dargestellt.

Das Ausführungsbeispiel zeigt ein optisches Abbildungssystems 1 zur Beobachtung eines Auges 2. Das optische Abbildungssystem 1 ist als stereoskopisches Beobachtungssystem mit einem rechten Beobachtungsstrahlengang 30 und einem linken Beobachtungsstrahlengang 40 ausgelegt und weist ein Hauptobjektiv 20 mit einer optischen Achse 23, eine rechte Tubuslinse 34, eine linke Tubuslinse 44, sowie ein rechtes Okular 36 und ein linkes Okular 46 auf. Es kann weitere, nicht dargestellte, Optik-Elemente umfassen.

Zwischen dem Hauptobjektiv 20 und dem Auge 2 ist ein weiteres optisches Element in Form einer Ophthalmoskopierlupe 3 und eine Reduzieroptik in Form von zwei Linsen in den Strahlengang eingebracht. Eine direkt vor dem Hauptobjektiv 20 angeordnete erste Linse der Reduzieroptik ist als eine Sammellinse 22 ausgeführt und weist eine positive Brechkraft auf. Eine zweite Linse der Reduzieroptik ist als eine Linse mit negativer Brechkraft, als eine Zerstreuungslinse 21 ausgeführt.

Der rechte Beobachtungsstrahlengang 30 und der linke eobachtungstrahlengang 40 durchsetzen die Ophthalmoskopierlupe 3 und können sich in einer Bildebene 10 kreuzen. Der von der Bildebene 10 ausgehende rechte Beobachtungsstrahlengang 30 durchdringt die Zerstreuungslinse 21, die Sammellinse 22, das am Hauptobjektiv 20, die rechte Tubuslinse 34 und gelangt zu dem rechten Okular 36. Dabei wird in dem rechten Beobachtungsstrahlengang 30 in einer rechten Okular-Zwischenbildebene 35 ein Okularzwischenbild erzeugt, das durch das rechte Okular 36 durch einen Beobachter betrachtet werden kann. Der von der Bildebene 10 ausgehende linke Beobachtungsstrahlengang 40 ist durch die Zerstreuungslinse 21, die Sammellinse 22, das Hauptobjektiv 20, die linke Tubuslinse 44 zu dem linken Okular 46 geführt. Dabei wird in dem linken Beobachtungsstrahlengang in einer linken Okular-Zwischenbildebene 45 ein Okularzwischenbild erzeugt, das durch das linke Okular 46 durch einen Beobachter betrachtet werden kann.

Der rechte Beobachtungsstrahlengang 30 verläuft nach dem Hauptobjektiv 20 parallel zu der optischen Achse 23. Dieser parallele Abstand ist mit gekennzeichnet. Entsprechend verläuft der linke Beobachtungsstrahlengang 40 nach dem Hauptobjektiv 20 ebenso in einem Abstand B parallel zu der optischen Achse 23. Der parallele Abstand der beiden eobachtungsstrahlengänge 30, 40 nach dem Hauptobjektiv 20 kann auch als Stereobasis SB bezeichnet werden, wobei die Stereobasis SB einen Wert aufweist, der doppelt so groß ist wie der Abstand B. Ein typischer Zahlenwert für die Stereobasis SB kann 25mm sein.

Typische Werte für die Brennweiten der Ophthalmoskopierlupe 3 sind 60 Dioptrien, 90 Dioptrien oder 120 Dioptrien. Die kurze Brennweite der Ophthalmoskopierlupe 3 kann eine Ursache für auftretende Bildfehler sein, die als spektrale Verkippung der optischen Achsen oder als eine chromatische Winkelabweichung CWA bezeichnet werden.

In Figur 1 sind schematisch die Auswirkungen der chromatischen Winkelabweichung CWA dargestellt. Unter der chromatischen Winkelabweichung CWA ist ein farbabhängiger chromatischer Bildversatz der Okularzwischenbilder, senkrecht zur optischen Achse 23, zu verstehen.

Für den rechten Beobachtungsstrahlengang 30 und den linken Beobachtungsstrahlengang 40 sind, bedingt durch einen symmetrischen Aufbau, jeweils vom Betrag her gleiche Werte für die chromatische Winkelabweichung CWA zu erwarten. Eine chromatische Winkelabweichung kann auch für einen einzelnen Beobachtungsstrahlengang auftreten. Für den rechten Beobachtungsstrahlengang 30 sind chromatische Winkelabweichungen für drei Wellenlängenbereiche in Form von drei Teilstrahlen dargestellt: Ein rechter roter Teilstrahl 31, ein rechter grüner Teilstrahl 32 und ein rechter blauer Teilstrahl 33. Für den linken Beobachtungsstrahlengang 40 ist eine chromatische Winkelabweichung für drei Wellenbereiche in Form eines linken roten Teilstrahls 41, eines linken grünen Teilstrahls 42 und eines linken blauen Teilstrahls 43 dargestellt. Der Bildversatz der rechten Teilstrahlen 31, 32, 33 im rechten Beobachtungsstrahlengang 30 ist in der rechten Okular-Zwischenbildebene 35 durch das rechte Okular 36 sichtbar. Der Bildversatz der linken Teilstrahlen 41, 42, 43 im linken Beobachtungsstrahlengang 40 ist in der linken Okular-Zwischenbildebene 45 durch das linke Okular 46 zu sehen.

Der Bildversatz, bedingt durch die chromatische Winkelabweichung CWA, wird ohne chromatische Korrektur als kontrastmindernd und störend empfunden. Liegt dieser chromatische Bildversatz über der Auflösungsgrenze des Auges, nimmt der Betrachter farbige Doppelbilder wahr. Der grüne Wellenlängenbereich, in Form der grünen Teilstrahlen 32, 42 ist im Zentrum der Okulare dargestellt. Der blaue Bildbereich in Form der blauen Teilstrahlen 33, 43 ist innerhalb des Bereichs zwischen den beiden Okularzentren sichtbar. Der rote Wellenlängenbereich, in Form der roten Teilstrahlen 31,41, ist außerhalb des Bereichs zwischen den Okularzentren sichtbar.

In diesem Ausführungsbeispiel ist die Reduzieroptik, umfassend die Sammellinse 22 und die Zerstreuungslinse 21, sehr nahe an dem Hauptobjektiv 20 angeordnet. Das Hauptobjektiv 20 weist eine Baulänge $L_{HO}$ auf. Als Baulänge $L_{HO}$ wird die Dicke oder Ausdehnung des Hauptobjektivs 20 entlang der der optischen Achse 23 bezeichnet. Eine gesamte Baulänge $L_{ges}$ bezeichnet die größte Ausdehnung des auptobjektivs 20 und der Reduzieroptik, umfassend die Zerstreuungslinse 21 und die Sammellinse 22, bezüglich der optischen Achse 23. Weist das Hauptobjektiv 20 beispielsweise eine Baulänge $L_{HO}$ = 1cm auf, so ergibt sich für die gesamte Baulänge Lges < 3,2cm.

Bedingt durch die sehr nahe Anordnung der Reduzieroptik vor dem Hauptobjektiv 20 stellt die Bereitstellung eines kontrastreichen, chromatisch korrigierten Bildbereichs mit sehr guter Abbildungsqualität eine besondere Herausforderung dar.

Es sei noch darauf verwiesen, dass die in diesem Ausführungsbeispiel gezeigte Bildebene 10 als Zwischenbildebene dargestellt ist, in der sich der rechte Beobachtungsstrahlengang 30 und der linke Beobachtungsstrahlengang kreuzen. Die Bildebene 10 kann auch eine andere zu beobachtende Objektebene darstellen. Ebenso kann die Position der Zerstreuungslinse 21 und die Position der Sammellinse 22 vertauscht angeordnet sein.

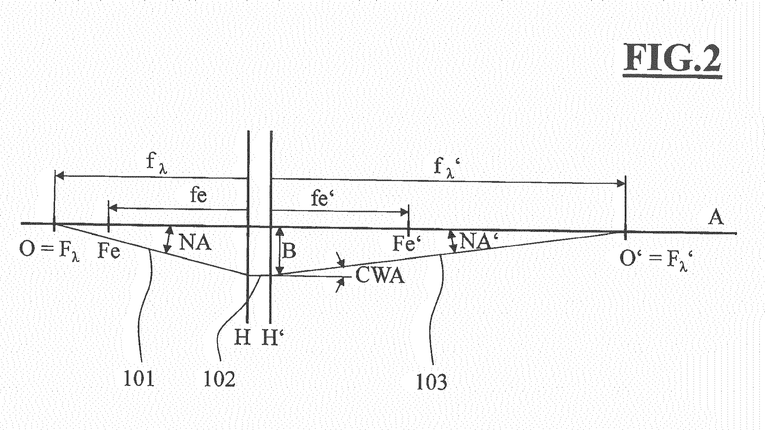

In Figur 2 zeigt eine schematische Darstellung eines Strahlengangs des optischen Abbildungssystems 1 gemäß Figur 1.

Eine optische Achse A ist als dicke horizontale Line gezeichnet. Senkrecht zu der optischen Achse A befindet sich eine erste Hauptebene H als Bezugsebene für Brennweiten oder Abstandsangaben im Objektraum und eine zweite Hauptebene H' als Bezugsebene für den Bildraum. Die beiden Hauptebenen H, H' erlauben es, die Wirkung des komplexen optischen Linsensystems mit der eine dünne Linse gültigen Gleichung zu beschreiben. Die erste Hauptebene H und die zweite Hauptebene H' sind beide senkrecht zur optischen Achse des Linsensyste definiert und verlaufen damit parallel zueinander. Die beiden Hauptebenen H, H' ersetzen das Hauptobjektiv 20 sowie die Reduzieroptik, umfassend die Zerstreuungslinse 21 und die Sammellinse 22.

Ein Objektpunkt O wird durch das optische Linsensystem zu einem Bildpunkt O' abgebildet. Von dem Objektpunkt O verläuft ein erster Strahl 101 in einem Winkel NA bezüglich der optischen Achse A zu der ersten Hauptebene H. Der Winkel NA wird auch als objektseitige numerische Apertur bezeichnet. Zwischen der ersten Hauptebene und der zweiten Hauptebene H' verläuft ein zweiter Strahl 102 parallel zu der optischen Achse A. Der zweite Strahl 102 hat in der ersten Hauptebene H und in der zweiten Hauptebene H' jeweils den Abstand B zur optischen Achse A. Bei einem stereoskopischen Abbildungssystem kann B einen Wert aufweisen, der vom Betrag her dem Zahlenwert der halben Stereobasis SB entspricht. Beim Austritt aus der zweiten Hauptebene H' ist ein dritter Strahl 103 zum Bildpunkt 0' gerichtet. Der dritte Strahl 103 schließt einen Winkel CWA bezüglich der optischen Achse A ein. Der Winkel CWA stellt die bildseitige chromatische Winkelabweichung CWA dar. Ist die bildseitige chromatische Winkelabweichung CWA gleich null, dann liegt der Bildpunkt O' im Unendlichen.

Für den Objektraum hat ein objektseitiger Brennpunkt Fe die objektseitige Brennweite fe für eine Hauptwellenlänge e. Bildraum hat der bildseitige Brennpunkt Fe' die bildseitige Brennweite fe' für die Hauptwellenlänge e. Für eine Wellenlänge $\lambda$ ergeben sich damit konform eine objektseitige Brennweite $f\lambda$ für einen objektseitigen Brennpunkt 0 = F$\lambda$, sowie eine bildseitige Brennweite $f\lambda$' für einen bildseitigen Brennpunkt O' = F$\lambda$'.

Zur Lösung der Aufgabe hat sich herausgestellt, dass die Maßzahl der chromatischen Winkelabweichung CWA eine sehr gute Möglichkeit darstellt, Maßnahmen zur Optimierung der Bildqualität zu bewerten. Sinnvollerweise geht man bei der Berechnung der Optik von einer Hauptwellenlänge e = 546nm aus. Die Hauptwellenlänge e wird auch als Fraunhofer-Linie e bezeichnet und definiert die Hauptwellenlänge im grünen Spektralbereich der Sonne. Um einen Objektpunkt O, beispielsweise die Bildebene 10, kontraststark und mit sehr guter Abbildungsqualität abzubilden, ist es notwendig, eine Optik bereitzustellen, die über den sichtbaren Wellenlängenbereich $\lambda$ zwischen 480nm und 660nm chromatisch korrigiert ist.

Vor dem Hauptobjektiv 20 ist die Reduzieroptik angeordnet, die die aus unterschiedlichen Materialien gefertigte Zerstreuungslinse 21 und Sammellinse 22, umfasst. Zum Erreichen einer guten Bildqualität wird für die Zerstreuungslinse 21 ein Material mit hoher Dispersion, d. h. niedriger Abbezahl gewählt. Die Sammellinse 22 weist ein Material mit niedriger Dispersion, d. h. hoher Abbezahl auf. Dabei liegt die Differenz der beiden Abbezahlen vorzugsweise zwischen 16 und 22. Vorzugsweise sind sowohl die Zerstreuungslinse 21 als auch die Sammellinse 22 aus einem Material mit hoher Brechzahl gefertigt, bevorzugt mit einer Brechzahl größer oder gleich 1,6. Durch Verwendung von Linsen mit hoher Brechzahl lassen sich gleichzeitig notwendige Korrekturen der monochromatischen Bildfehler, wie sphärische Aberration, Koma oder Astigmatismus korrigieren. Eine zusätzliche Korrektur der monochromatischen Bildfehler lässt sich erreichen, wenn die Sammellinse 22 und die Zerstreuungslinse 21 annähernd gleiche Brennweiten, jedoch mit verschiedenen Vorzeichen aufweisen.

Eine sehr gute, kontrastreiche Bildqualität ist erreicht, wenn die Farbbereiche der rechten Teilstrahlen 31, 32, 33 des rechten Beobachtungsstrahlengangs 30, sowie die Farbbereiche der linken Teilstrahlen 41, 42, 43 des linken Beobachtungsstrahlengangs jeweils in der Okular-Zwischenbildebene 35, 45 als deckungsgleich empfunden werden.

Dazu ist es notwendig, dass für alle Wellenlängenbereiche des sichtbaren Lichts zwischen 480nm und 660nm ein chromatische Winkelabweichung erreicht ist, die kleiner ist als 0,5'.

Wie in Figur 2 gezeigt, sind die objektseitigen und bildseitigen Brennweiten des optischen Systems bezüglich der beiden Hauptebenen H und H' jeweils abhängig von der Wellenlänge $\lambda$. Dabei sind die folgenden drei Brennweiten von Bedeutung:

fe = objektseitige Brennweite für die Hauptwellenlänge e bezüglich der ersten Hauptebene H;

f$\lambda$ = objektseitige Brennweite für die Wellenlänge $\lambda$ bezüglich der ersten Hauptebene H;

fe' = bildseitige Brennweite für die Hauptwellenlänge e bezüglich der zweiten Hauptebene H'.

Ist das Linsensystem derart ausgestaltet, d. h. die Reduzieroptik, umfassend die Zerstreuungslinse 21 und die Sammellinse 22, sowie das Hauptobjektiv 20 derart aufeinander abgestimmt, dass für einen Wellenlängenbereich $\lambda$ von 480nm $\leq \lambda \leq$ 660nm und für eine Hauptwellenlänge e = 546nm folgende Beziehung erfüllt ist,

$$\left| \arctan\left( \frac{B}{fe' + (fe \cdot fe'/(f\lambda - fe))} \right) \right| < 0.5',$$

so ist die Abbildungsqualität für einen Beobachtungsstrahlengang so gut korrigiert, dass ein gleichbleibender guter Kontrast der Abbildung und eine gleichbleibende, sehr gute Bildqualität über den gesamten Wellenlängenbereich $\lambda$ erreicht werden. Damit werden die Farbbereiche der rechten Teilstrahlen 31, 32, 33 des rechten Beobachtungsstrahlengangs 30, sowie die Farbbereiche der linken Teilstrahlen 41, 42, 43 des linken Beobachtungsstrahlengangs in der Okular-Zwischenbildebene 35, 45 jeweils als deckungsgleich empfunden. Damit ist eine chromatische Winkelabweichung erreicht, die kleiner ist als 0,5'.

Beispielsweise lässt sich für eine Wellenlänge $\lambda$ = 660nm die chromatische Winkelabweichung CWA für das Ausführungsbeispiel wie folgt berechnen:

fe = -175,102mm (für e = 546nm)

fe' = +175,102mm (für e = 546nm)

f$\lambda$ = -175,033mm (für $\Delta$ = 660nm)

B = 12mm

Damit ergibt sich für die CWA (in Winkelminuten):

$$CWA = \left| \arctan\left( \frac{B}{fe' + (fe \cdot fe'/(f\lambda - fe))} \right) \right|$$

$$= \left| \arctan\left( \frac{12mm}{175{,}102mm + (-175{,}102mm \cdot 175{,}102mm/(-175{,}033mm - (-175{,}102mm)))} \right) \right.$$

$$= 0{,}093'$$

Da 0,093' < 0,5' ist die Bedingung für eine gute Bildqualität bei einer geringen chromatischen Winkelabweichung CWA für eine Wellenlänge $\lambda$ = 660nm und eine Hauptwellenlänge e = 546nm erfüllt.

Ist diese Bedingung für alle Wellenlängen $\lambda$ zwischen 480nm und 660nm erfüllt, so ist die gewählte Material- und Form-Kombination für die Zerstreuungslinse 21 und die Sammellinse 22 der Reduzieroptik geeignet, die Aufgabe zu erfüllen.

Beim Einbringen der Reduzieroptik in die Beobachtungsstrahlengänge 30, 40 ist es wünschenswert, dass eine Veränderung der Fokuseinstellung des Mikroskops nicht notwendig ist. Dazu ist es von Vorteil, wenn eine Brennweitenänderung, d. h. eine Fokussierung auf die Bildebene 10, durch die Reduzieroptik vorgenommen werden kann. Im Ausführungsbeispiel in Figur 1 ist die gesamte Brennweite $F_{ges}$ des optischen Systems, umfassend das Hauptobjektiv 20 und die Reduzieroptik, so gewählt, dass diese im Bereich zwischen dem 0,7-fachen und dem 1,1-fachen der Brennweite des Hauptobjektivs $F_{HO}$ liegt. Dieser Wert ist ausreichend, um eine Fokussierung durch die Reduzieroptik auf die Bildebene 10 zu ermöglichen. Dazu ist die Zerstreuungslinse 21 fest angeordnet und die Sammellinse 22 verschiebbar entlang der optischen Achse 23 angeordnet, wie durch den Doppelpfeil oberhalb der Sammellinse 22 in Figur 1 angedeutet ist. In einer alternativen Ausführungsform kann auch die Sammellinse 22 fest angeordnet und die Zerstreuungslinse 21 verschiebbar entlang der optischen Achse 23 angeordnet sein. Die Figur 1 zeigt eine mittlere Fokuseinstellung mit einer Brennweite $F_{ges} = 0.87 * F_{HO}$.

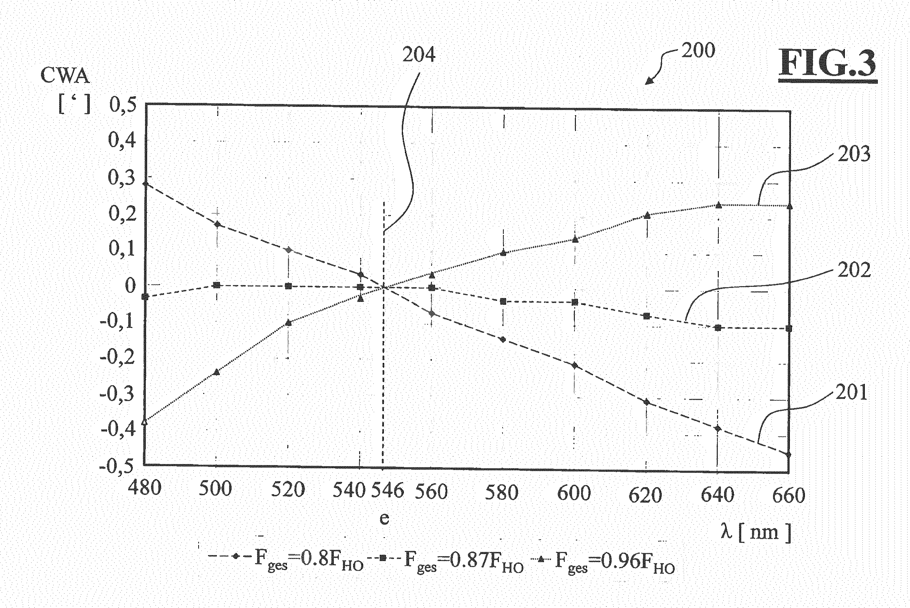

Ein Ergebnis für eine geeignete Material- und Formfestlegung für die Zerstreuungslinse 21 und die Sammellinse 22, die für die Wellenlängen des sichtbaren Lichts und verschiedenen Fokuseinstellungen ein chromatisch korrigiertes, kontrastreiches Bild liefert, zeigt das Diagramm in Figur 3.

In Figur 3 ist eine bildseitige chromatische Winkelabweichung CWA bezogen auf eine Wellenlänge $\lambda$ im sichtbaren Bereich zwischen 480nm und 660nm für drei unterschiedliche Brennweiten des ersten Ausführungsbeispiels dargestellt.

Das Diagramm 200 zeigt die chromatische Winkelabweichung CWA in Winkelminuten auf der Y-Achse in einem Bereich von -0,5' bis +0,5'. Auf der X-Achse ist der Wellenlängenbereich $\lambda$ zwischen 480nm und 660nm dargestellt. Die Hauptwellenlänge e = 546nm ist durch eine gestrichelte Linie 204 hervorgehoben. Die chromatische Winkelabweichung CWA ist für drei Brennweiten dargestellt: Eine erste Brennweite $F_{ges} = 0,80 * F_{HO}$ ist in einer ersten Kurve 201 gezeigt; eine zweite Brennweite $F_{ges} = 0,87 * F_{HO}$ ist in einer zweiten Kurve 202 dargestellt und eine dritte Brennweite $F_{ges} = 0,96 * F_{HO}$ ist in einer dritten Kurve 203 gezeigt. Die erste Kurven 201 und die dritte Kurve 203 zeigen die Fokuseinstellung jeweils in einer möglichen Endlagenposition.

Für die mittlere Fokuseinstellung mit einer Brennweite $F_{ges} = 0.87 * F_{HO}$ ist für den gesamten Wellenlängenbereich $\lambda$ zwischen 480nm und 660nm die bildseitige chromatische Winkelabweichung CWA besonders gut korrigiert und liegt im Bereich zwischen -0,1' und null, siehe zweite Kurve 202. Für die Hauptwellenlänge e = 546nm ist chromatische Winkelabweichung CWA gleich null.

Die chromatische Winkelabweichung CWA für eine Fokuseinstellung mit der Brennweite $F_{ges} = 0.80 * F_{HO}$ liegt für den gesamten Wellenlängenbereich $\lambda$ zwischen 480nm und 660nm im Bereich zwischen +0,28' und -0,45', siehe erste Kurve 201. Auch in dieser Fokuseinstellung ist für die Hauptwellenlänge e = 546nm die chromatische Winkelabweichung CWA gleich null.

Die bildseitige chromatische Winkelabweichung CWA für eine Fokuseinstellung mit der Brennweite $F_{ges} = 0.97 * F_{HO}$ liegt für den gesamten Wellenlängenbereich $\lambda$ zwischen 480nm und 660nm im Bereich zwischen -0,39' und +0,24', siehe dritte Kurve 203. Auch in dieser Fokuseinstellung ist für die Hauptwellenlänge e = 546nm die bildseitige chromatische Winkelabweichung CWA gleich null.

Die Kurven 201, 202 und 203 zeigen deutlich, dass die bildseitige chromatische Winkelabweichung CWA über den gesamten Fokussierbereich für alle Brennweiten im Bereich von +/- 0,5' liegt.

Damit ist ein optisches Abbildungssystem 1 bereitgestellt, das unter Verwendung einer in einen Strahlengang 30, 40 einbringbaren fokussierbaren Reduzieroptik, umfassend eine Zerstreuungslinse 21 und eine Sammellinse 22, eine sehr geringen Baulänge vor einem Hauptobjektiv erreicht. Diese optische Abbildungssystem erreicht eine sehr gute Abbildungsqualität bei einer äußerst geringen chromatische Winkelabweichung über den gesamten Fokussierbereich.

**Bezugszeichenliste**

| | |
|---|---|
| 1 | Optisches Abbildungssystem |
| 2 | Auge |
| 3 | Ophthalmoskopierlupe |
| 10 | Bildebene |
| 20 | Hauptobjektiv |
| 21 | Zerstreuungslinse |
| 22 | Sammellinse |
| 23 | Optische Achse |
| 30 | Rechter Beobachtungsstrahlengang |
| 31 | Rechter roter Teilstrahl |
| 32 | Rechter grüner Teilstrahl |
| 33 | Rechter blauer Teilstrahl |
| 34 | Rechte Tubuslinse |
| 35 | Rechte Okular-Zwischenbildebene |
| 40 | Linker Beobachtungsstrahlengang |
| 41 | Linker roter Teilstrahl |
| 42 | Linker grüner Teilstrahl |
| 43 | Linker blauer Teilstrahl |
| 44 | Linke Tubuslinse |
| 45 | Linke Okular-Zwischenbildebene |

101 Erster Strahl des Strahlengangs vom Objektpunkt O zu Hauptebene H im Objektraum
102 Zweiter Strahl des Strahlengangs zwischen den Hauptebenen H und H'
103 Dritter Strahl des Strahlengangs von der Hauptebene H' zum Bildpunkt O' im Bildraum
200 Diagramm chromatische Winkelabweichung CWA
201 Erste Kurve, CWA für $F_{ges}$ 0.80$F_{HO}$
202 Zweite Kurve, CWA für $F_{ges}$= 0.87$F_{HO}$
203 Dritte Kurve, CWA für $F_{ges}$ 0.96$F_{HO}$

**Patentansprüche**

1. Optisches Abbildungssystem (1) zur Erzeugung eines Bildes einer Objektebene mit einem Linsensystem, das ein Hauptobjektiv (20) und eine Reduzieroptik zwischen dem Hauptobjektiv (20) und der Objektebene umfasst und das entlang einer optischen Achse (23) ausgerichtet ist, wobei die Reduzieroptik eine erste Linse (22) mit einer positiven Brechkraft und eine zweite Linse (21) mit einer negativen Brechkraft aufweist,
wobei eine objektseitige erste Hauptebene (H) und eine bildseitige zweite Hauptebene (H') durch das Linsensystem definiert ist,
wobei durch das optische Abbildungssystem (1) ein Beobachtungsstrahlengang (30, 40) definiert ist, der derart durch das Linsensystem geführt ist, dass der Beobachtungsstrahlengang (30, 40) in der ersten Hauptebene (H) und in der zweiten Hauptebene (H') jeweils einen Abstand B zur optischen Achse (23) des Linsensystems aufweist,
**dadurch kennzeichnet, dass**
die erste Linse (22) aus einem ersten Material gefertigt ist, das eine erste Abbezahl aufweist, und dass die zweite Linse (21) aus einem zweiten Material gefertigt ist, das eine zweite Abbezahl aufweist, wobei die erste Abbezahl größer ist als die zweite Abbezahl,
und dass das Linsensystem derart ausgestaltet ist, dass für einen Wellenlängenbereich λ von 480nm ≤ λ ≤ 660nm und für eine Hauptwellenlänge e = 546nm folgende Beziehung erfüllt ist:

$$\left| \arctan\left( \frac{B}{f_{e'} + (f_e \cdot f_{e'} / (f_\lambda - f_e))} \right) \right| < 0.5',$$

wobei gilt:

$f_e$ = objektseitige Brennweite für die Hauptwellenlänge e bezüglich der ersten Hauptebene (H);
$f_\lambda$ = objektseitige Brennweite für die Wellenlänge λ bezüglich der ersten Hauptebene (H);
$f_{e'}$ = bildseitige Brennweite für die Hauptwellenlänge e bezüglich der zweiten Hauptebene (H').

2. Optisches Abbildungssystem nach Anspruch 1,
**dadurch kennzeichnet, dass**
das erste Material und das zweite Material derart gewählt sind, dass eine Differenz der ersten Abbezahl und der zweiten Abbezahl zwischen 16 und 22 liegt.

3. Optisches Abbildungssystem nach Anspruch 1 oder 2,
**dadurch kennzeichnet, dass**
das erste Material und das zweite Material derart gewählt sind, dass eine erste Brechzahl des ersten Materials größer ist als 1,6 und eine zweite Brechzahl des zweiten Materials größer ist als 1,6.

4. Optisches Abbildungssystem nach einem der Ansprüche 1bis 3,
**dadurch kennzeichnet, dass**
die erste Linse (22) fest angeordnet ist und die zweite Linse (21) verschiebbar in Richtung der optischen Achse angeordnet ist.

5. Optisches Abbildungssystem nach einem der Ansprüche 1 bis 3,
**dadurch kennzeichnet, dass**
die zweite Linse (21) fest angeordnet ist und die erste Linse (22) verschiebbar in Richtung der optischen Achse angeordnet ist.

**6.** Optisches Abbildungssystem nach einem der vorherigen Ansprüche,
**dadurch kennzeichnet, dass**
dass die Reduzieroptik in den Strahlengang vor dem Hauptobjektiv (20) einschwenkbar ist.

**7.** Optisches Abbildungssystem nach einem der vorherigen Ansprüche,
**dadurch kennzeichnet, dass**
das vor der Reduzieroptik ein weiteres optisches Element (3) zur Erzeugung eines Zwischenbildes (10) im Beobachtungsstrahlengang angebracht ist und dass das optische Abbildungssystem (1) auf das Zwischenbild (10) fokussiert ist.

**8.** Optisches Abbildungssystem nach einem der vorherigen Ansprüche,
**dadurch kennzeichnet, dass**
das optische Abbildungssystem (1) als ein Stereomikroskop ausgebildet ist und einen ersten Beobachtungsstrahlengang (30) und einen zweiten Beobachtungsstrahlengang (40) aufweist, wobei der erste und der zweite Beobachtungsstrahlengang (30, 40) in der ersten Hauptebene (H) und in der zweiten Hauptebene (H') jeweils einen Abstand B zur optischen Achse des Linsensystems aufweisen.

FIG.1

FIG.2

FIG.3

Europäisches
Patentamt

European
Patent Office

Office européen
des brevets

**EUROPÄISCHER RECHERCHENBERICHT**

Nummer der Anmeldung

EP 14 18 4931

| | EINSCHLÄGIGE DOKUMENTE | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG   (IPC) |
| X | EP 1 326 117 A1 (ZEISS CARL [DE]; ZEISS STIFTUNG [DE]) 9. Juli 2003 (2003-07-09) * Absatz [0044] - Absatz [0049]; Abbildung 7 * ----- | 1-8 | INV. G02B21/02 G02B21/22 |
| A | CH 663 284 A5 (ZEISS CARL FA) 30. November 1987 (1987-11-30) * Seite 2, linke Spalte, Zeile 67 - Seite 4, rechte Spalte, Zeile 64; Abbildungen 1, 3, 5, 6; Tabellen II, III * ----- | 1,8 | |
| A | DE 10 2010 018123 A1 (ZEISS CARL MICROIMAGING GMBH [DE]) 27. Oktober 2011 (2011-10-27) * Absatz [0047] - Absatz [0054]; Abbildungen 5, 6 * * Absatz [0006] - Absatz [0008] * ----- | 1,8 | |

| RECHERCHIERTE SACHGEBIETE   (IPC) |
|---|
| G02B A61B |

Der vorliegende Recherchenbericht wurde für alle Patentansprüche erstellt

| Recherchenort | Abschlußdatum der Recherche | Prüfer |
|---|---|---|
| Berlin | 17. Februar 2015 | A. Jacobs |

KATEGORIE DER GENANNTEN DOKUMENTE

X : von besonderer Bedeutung allein betrachtet
Y : von besonderer Bedeutung in Verbindung mit einer
  anderen Veröffentlichung derselben Kategorie
A : technologischer Hintergrund
O : nichtschriftliche Offenbarung
P : Zwischenliteratur

T : der Erfindung zugrunde liegende Theorien oder  Grundsätze
E : älteres Patentdokument, das jedoch erst am oder
  nach dem Anmeldedatum veröffentlicht worden ist
D : in der Anmeldung angeführtes Dokument
L : aus anderen Gründen angeführtes Dokument
...................................................................................
& : Mitglied der gleichen Patentfamilie, übereinstimmendes
  Dokument

EPO FORM 1503 03.82 (P04C03)

**ANHANG ZUM EUROPÄISCHEN RECHERCHENBERICHT**
**ÜBER DIE EUROPÄISCHE PATENTANMELDUNG NR.**            EP 14 18 4931

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

17-02-2015

| Im Recherchenbericht angeführtes Patentdokument | Datum der Veröffentlichung | Mitglied(er) der Patentfamilie | Datum der Veröffentlichung |
|---|---|---|---|
| EP 1326117 A1 | 09-07-2003 | AT 332509 T | 15-07-2006 |
| | | AT 369579 T | 15-08-2007 |
| | | DE 10140402 A1 | 11-04-2002 |
| | | DE 10146971 A1 | 11-04-2002 |
| | | EP 1191381 A2 | 27-03-2002 |
| | | EP 1293819 A1 | 19-03-2003 |
| | | EP 1326117 A1 | 09-07-2003 |
| | | EP 1770427 A2 | 04-04-2007 |
| | | ES 2291258 T3 | 01-03-2008 |
| | | JP 2002174773 A | 21-06-2002 |
| | | US 2002075449 A1 | 20-06-2002 |
| CH 663284 A5 | 30-11-1987 | CA 1244685 A1 | 15-11-1988 |
| | | CH 663284 A5 | 30-11-1987 |
| | | DE 3217776 A1 | 17-11-1983 |
| | | GB 2120402 A | 30-11-1983 |
| | | JP S58203411 A | 26-11-1983 |
| | | MX 152710 A | 18-10-1985 |
| | | US 4518231 A | 21-05-1985 |
| DE 102010018123 A1 | 27-10-2011 | KEINE | |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 1227355 B1